# EUROPEAN PATENT SPECIFICATION

(11) **EP 3 155 146 B1**
(45) Date of publication and mention of the grant of the patent: **23.06.2021**
(21) Application number: 15810606.2
(22) Date of filing: 18.05.2015
(51) Int. Cl.: C40B 20/04, C40B 30/04, C40B 70/00, C12Q 1/68, C12P 19/34, C12Q 1/6837

(54) **HIGH THROUGHPUT GENE ASSEMBLY IN DROPLETS**
HOCHDURCHSATZGENASSEMBLIERUNG IN TRÖPFCHEN
ASSEMBLAGE DE GÈNES À HAUT DÉBIT DANS DES GOUTTELETTES

(30) Priority: 16.06.2014 US 201462012842 P; 15.09.2014 US 201414486346; 21.11.2014 US 201414550713
(43) Date of publication of application: 19.04.2017
(73) Proprietor: Agilent Technologies, Inc., Santa Clara, CA 95051-7201 (US)
(72) Inventor: SAMPSON, Jeffrey, R., Santa Clara, CA 95051-7201 (US); SAMPAS, Nicholas, M., Santa Clara, CA 95051-7201 (US); MYERSON, Joel, Santa Clara, CA 95051-7201 (US); ANDERSON, Paige, Santa Clara, CA 95051-7201 (US); CURRY, Bo, Santa Clara, CA 95051-7201 (US)
(74) Representative: Grünecker Patent- und Rechtsanwälte PartG mbB
(86) International application number: PCT/US2015/031412
(87) International publication number: WO 2015/195257

(56) References cited:
- WO-A1-2007/040592
- WO-A1-2012/154201
- WO-A1-2015/175832
- WO-A2-2008/054543
- WO-A2-2009/109753
- WO-A2-2010/025310
- WO-A2-2011/056872
- WO-A2-2014/092886
- US-A1- 2010 113 296
- US-A1- 2010 113 296
- US-A1- 2010 291 578
- US-A1- 2014 045 728
- SIYING MA ET AL: "DNA synthesis, assembly and applications in synthetic biology", CURRENT OPINION IN CHEMICAL BIOLOGY, vol. 16, no. 3-4, 1 August 2012 (2012-08-01), pages 260-267, XP055212288, ISSN: 1367-5931, DOI: 10.1016/j.cbpa.2012.05.001
- XIONG ET AL: "Non-polymerase-cycling-assembly-based chemical gene synthesis: Strategies, methods, and progress", BIOTECHNOLOGY ADVANCES, ELSEVIER PUBLISHING, BARKING, GB, vol. 26, no. 2, 7 November 2007 (2007-11-07), pages 121-134, XP022426820, ISSN: 0734-9750, DOI: 10.1016/J.BIOTECHADV.2007.10.001
- AI-SHENG XIONG ET AL: "Chemical gene synthesis: strategies, softwares, error corrections, and applications", FEMS MICROBIOLOGY REVIEWS, vol. 32, no. 3, 1 May 2008 (2008-05-01), pages 522-540, XP055439903, DOI: 10.1111/j.1574-6976.2008.00109.x
- GIBSON DANIEL G: "Enzymatic assembly of overlapping DNA fragments", METHODS IN ENZYMO, ACADEM. PRESS, USA, vol. 498, 1 January 2011 (2011-01-01), pages 349-361, XP009179862, ISSN: 1557-7988

## Description

### BACKGROUND

High-throughput synthesis and assembly of DNA constructs is an integral part of synthetic biology and the bio-engineering cycle which aims to revolutionize how molecular and biological products are developed and manufactured. A number of methods for the assembly of synthetic DNA oligonucleotides into longer constructs have been developed over the past several years. Many methods utilize a combination of polymerase or ligase enzymes to join shorter oligonucleotides (e.g., molecules that are 50 to 200 nucleotides in length) to form constructs that are as long as 1,000 to 5,000 base-pairs. These methods are sufficient for the construction of whole genes coding for functional proteins.

Many high throughput methods are performed in micro-titer plates using automated robotic systems. While these systems reduce the cost of labor, the reagent costs, including the starting oligonucleotides, are still considerable given the number and the volume of the various reactions required for the assembly.

### SUMMARY

This disclosure provides, among other things, a method comprising: (a) obtaining a mixture of multiple sets of oligonucleotides, wherein the oligonucleotides within each set each comprise a terminal indexer sequence and can be assembled to produce a synthon; and (b) hybridizing the oligonucleotide mixture to an array, thereby spatially-separating the different sets of oligonucleotides from one another. In some embodiments the method may comprise (c) contacting the array with a solution, thereby producing, for each feature bound by the oligonucleotides, a discrete droplet comprising the feature and, optionally, placing an immiscible liquid over the droplets, thereby producing, for each feature bound by the oligonucleotides, a discrete reaction chamber defined by a droplet. The method may further comprise incubating the array under conditions by which a synthon is assembled in each of the reaction chambers. Also provided is a composition comprising multiple sets of oligonucleotides, wherein the oligonucleotides within each set comprise a terminal indexer sequence and can be assembled to produce a synthon.

Other devices, apparatus, systems, methods, features and advantages of the invention will be or will become apparent to one with skill in the art upon examination of the following figures and detailed description. It is intended that all such additional systems, methods, features and advantages be included within this description and be within the scope of the invention.

### BRIEF DESCRIPTION OF THE FIGURES

The skilled artisan will understand that the drawings, described below, are for illustration purposes only. The drawings are not intended to limit the scope of the present teachings in any way.
Fig. 1 schematically illustrates some of the general principles of one embodiment of the subject method.
Fig. 2 schematically illustrates one embodiment of an oligonucleotide from an oligonucleotide library hybridized to an oligonucleotide on an array.
Fig. 3 schematically illustrates how the library oligonucleotide of Fig. 2 can be extended to produce a double stranded oligonucleotide.
Fig. 4 schematically illustrates how reaction vessels can be formed by dipping an array into an aqueous fluid.
Fig. 5 illustrates a second embodiment of an oligonucleotide from an oligonucleotide library hybridized to an oligonucleotide on an array.
Fig. 6 schematically illustrates how the library oligonucleotide of Fig. 5 can be extended to produce a double stranded extension product.
Fig. 7 schematically illustrates an alternative assembly method that uses double stranded oligonucleotides that have cleavable linkages.
Fig. 8 schematically illustrates an alternative assembly method that uses single stranded oligonucleotides.
Fig. 9 schematically illustrates a set of oligonucleotides that contain (i) a terminal indexer sequence and (ii) an assembly sequence, wherein the terminal indexer sequence and the assembly sequence are separated by a photocleavable or chemically cleavable linker.
Fig. 10 schematically illustrates an oligonucleotide that contains (i) a terminal indexer sequence and (ii) an assembly sequence, wherein the terminal indexer sequence and the assembly sequence are separated by a photocleavable or chemically cleavable linker, and hybridize to a feature of an array.
Figs. 11-14 schematically illustrate alternative methods by which assemblable fragments can be produced.

### DEFINITIONS

Before describing exemplary embodiments in greater detail, the following definitions are set forth to illustrate and define the meaning and scope of the terms used in the description.

Numeric ranges are inclusive of the numbers defining the range. Unless otherwise indicated, nucleic acids are written left to right in 5' to 3' orientation; amino acid sequences are written left to right in amino to carboxy orientation, respectively.

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Markham, THE HARPER COLLINS DICTIONARY OF BIOLOGY, Harper Perennial, N.Y. (1991) provide one of skill with the general meaning of many of the terms used herein. Still, certain terms are defined below for the sake of clarity and ease of reference.

It must be noted that as used herein and in the appended claims, the singular forms "a", "an", and "the" include plural referents unless the context clearly dictates otherwise. For example, the term "a primer" refers to one or more primers, i.e., a single primer and multiple primers. It is further noted that the claims can be drafted to exclude any optional element. As such, this statement is intended to serve as antecedent basis for use of such exclusive terminology as "solely," "only" and the like in connection with the recitation of claim elements, or use of a "negative" limitation.

As used herein, the term "array" is intended to describe a two-dimensional arrangement of addressable regions bearing oligonucleotides associated with that region. The oligonucleotides of an array may be covalently attached to substrate at any point along the nucleic acid chain, but are generally attached at one terminus (e.g. the 3' or 5' terminus).

Any given substrate may carry one, two, four or more arrays disposed on a front surface of the substrate. Depending upon the use, any or all of the arrays may be the same or different from one another and each may contain multiple spots or features. An array may contain at least 10, at least 100, at least 1,000, at least 10,000, at least 100,000, or at least 10⁶ or more features, in an area of less than 20 cm², e.g., in an area of less than 10 cm², of less than 5 cm², or of less than 1 cm². In some embodiments, features may have widths (that is, diameter, for a round spot) in the range from 1 µm to 1.0 cm, although features outside of these dimensions are envisioned. In some embodiments, a feature may have a width in the range of 3.0 µm to 200 µm, e.g., 5.0 µm to 100 µm or 10 µm to 50 µm. Interfeature areas will typically be present which do not carry any polymeric compound. It will be appreciated though, that the interfeature areas, when present, could be of various sizes and configurations.

Each array may cover an area of less than 100 cm², e.g., less than 50 cm², less than 10 cm² or less than 1 cm². In some embodiments, the substrate carrying the one or more arrays will be shaped generally as a rectangular or square solid (although other shapes are possible), having a length of more than 4 mm and less than 10 cm, e.g., more than 5 mm and less than 5 cm, and a width of more than 4 mm and less than 10 cm, e.g., more than 5 mm and less than 5 cm.

Arrays can be fabricated using drop deposition from pulse jets of either polynucleotide precursor units (such as monomers) in the case of *in situ* fabrication, or the previously obtained polynucleotide. Such methods are described in detail in, for example, US 6,242,266, US 6,232,072, US 6,180,351, US 6,171,797, US 6,323,043, U.S. Patent Application Serial No. 09/302,898 filed April 30, 1999 by Caren et al., and the references cited therein. Other drop deposition methods can be used for fabrication, as previously described herein. Also, instead of drop deposition methods, photolithographic array fabrication methods may be used. Interfeature areas need not be present particularly when the arrays are made by photolithographic methods.

An array is "addressable" when it has multiple regions of different moieties (e.g., different polynucleotide sequences) such that a region (i.e., a "feature", "spot" or "area" of the array) is at a particular predetermined location (i.e., an "address") on the array. Array features are typically, but need not be, separated by intervening spaces.

The term "nucleotide" is intended to include those moieties that contain not only the known purine and pyrimidine bases, but also other heterocyclic bases that have been modified. Such modifications include methylated purines or pyrimidines, acylated purines or pyrimidines, or other heterocycles. In addition, the term "nucleotide" includes those moieties that contain hapten or fluorescent labels and may contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The terms "nucleic acid" and "polynucleotide" are used interchangeably herein to describe a polymer of any length, e.g., greater than about 2 bases, greater than about 10 bases, greater than about 100 bases, greater than about 500 bases, greater than 1000 bases, up to about 10,000 or more bases composed of nucleotides, e.g., deoxyribonucleotides or ribonucleotides, and may be produced enzymatically or synthetically (e.g., PNA as described in U.S. Patent No. 5,948,902 and the references cited therein) and which can hybridize with naturally occurring nucleic acids in a sequence specific manner analogous to that of two naturally occurring nucleic acids, e.g., can participate in Watson-Crick base pairing interactions. Naturally-occurring nucleotides include guanine, cytosine, adenine, thymine, uracil (G, C, A, T and U respectively). DNA and RNA have a deoxyribose and ribose sugar backbone, respectively, whereas PNA's backbone is composed of repeating N-(2-aminoethyl)-glycine units linked by peptide bonds. In PNA various purine and pyrimidine bases are linked to the backbone by methylene carbonyl bonds. A locked nucleic acid (LNA), often referred to as an inaccessible RNA, is a modified RNA nucleotide. The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired. The term "unstructured nucleic acid", or "UNA", is a nucleic acid containing non-natural nucleotides that bind to each other with reduced stability. For example, an unstructured nucleic acid may contain a G' residue and a C' residue, where these residues correspond to non-naturally occurring forms, i.e., analogs, of G and C that base pair with each other with reduced stability, but retain an ability to base pair with naturally occurring C and G residues, respectively. Unstructured nucleic acid is described in US20050233340.

The term "oligonucleotide" as used herein denotes a multimer of nucleotide of from about 2 to 200 nucleotides, up to 500 nucleotides in length. Oligonucleotides may be synthetic or may be made enzymatically, and, in some embodiments, are 30 to 150 nucleotides in length. Oligonucleotides may contain ribonucleotide monomers (i.e., may be oligoribonucleotides) and/or deoxyribonucleotide monomers. An oligonucleotide may be 10 to 20, 21 to 30, 31 to 40, 41 to 50, 51-60, 61 to 70, 71 to 80, 80 to 100, 100 to 150 or 150 to 200 nucleotides in length, for example.

The term "primer" as used herein refers to an oligonucleotide, whether occurring naturally as in a purified restriction digest or produced synthetically, which is capable of acting as a point of initiation of synthesis when placed under conditions in which synthesis of a primer extension product, which is complementary to a nucleic acid strand, is induced, *i.e.,* in the presence of nucleotides and an inducing agent such as a DNA polymerase and at a suitable temperature and pH. A primer must be sufficiently long to prime the synthesis of the desired extension product in the presence of the inducing agent. The exact length of the primer will depend upon many factors, including temperature, source of primer and use of the method. For example, for some applications, depending on the complexity of the target sequence, the oligonucleotide primer may contain 15-25 or more nucleotides, although it may contain fewer nucleotides.

An array of polymeric compounds can be made using any suitable method, including methods in which pre-made polymeric compounds are deposited onto the surface of a substrate and then linked to the substrate, and also *in situ* synthesis methods.

As used herein, the term "areas that contain the polymeric compounds on the surface of the substrate" is intended to refer to the features that contain the polymeric compounds, as discussed above.

As used herein, the terms "hydrophobic" and "hydrophilic" are relative terms and are intended to refer to the degree by which a solution is attracted to or repelled from a surface. Hydrophobicity and hydrophilicity may be measured by measuring the contact angle of the solution on the surface, as described in Johnson et al. (J. Phys. Chem. 1964 Contact Angle Hysteresis 68: 1744-1750). Contact angle is a measure of static hydrophobicity, and contact angle hysteresis and slide angle are dynamic measures. See also the paper entitled Contact Angle Measurements Using the Drop Shape Method by Roger P. Woodward, which can be obtained at the website formed by placing "http://www." in front of "firsttenangstroms.com/pdfdocs/CAPaper.pdf".

As used herein, the term "selectively hydrating" is intended to refer to a step in which an aqueous solution is selectively applied to the areas of an array that contain the polymeric compounds (or selected groups thereof that are immediately adjacent to one another), but not the areas in between those areas. This step results in a substrate that has an array of droplets on its surface, where the edges of the droplets correspond to the boundaries of the features that contain the polymeric compounds. This may be done using a variety of methods, including dipping an array into an aqueous solution, where the difference in hydrophobicity between the features and non-feature areas results in droplets that are confined to the features.

As used herein, the term "discrete droplets" is intended to refer to droplets on the surface of the substrate that are separated from one another. Each discrete droplet may occupy a single feature of an array (i.e., where each droplet lies over a single polymeric compound) or each discrete droplet may occupy multiple features of an array (where the droplets are actively induced to bleed into each other in a pre-defined way so that one droplet can contain multiple oligonucleotides).

As used herein, the term "pre-defined" is intended to refer to something that is known prior to being made.

As used herein, the term "releasing the polymeric compounds from the surface" is intended to refer to a step in which products are cleaved from the substrate surface. This step is done by cleaving a cleavable linker that links the products to the surface of the array. This may be done using a photocleavable linker or a restriction enzyme, for example.

As used herein, the term "adjacent to one another on the substrate" is intended to refer to areas that contain polymeric compounds that are immediately adjacent to one another (i.e., next to each other, without any other areas that contain polymeric compounds that are in between).

As used herein, the term "mixture" is intended to refer to a solution in which the components are interspersed with one another and not spatially separated.

As used herein, the term "aqueous" is intended to refer to a medium in which the solvent comprises water.

As used herein, the terms "sets", "multiple" and "plurality" refer to a population that contains at least 2 members. In certain cases, a plurality may have at least 10, at least 100, at least 1,000, at least 10,000, at least 100,000, at least 106, at least 107, at least 108 or at least 109 or more members.

As used herein, the term "in the solution phase" is intended to refer to a polymeric compound that is in an aqueous environment that is not bound or tethered to a solid substrate. Such a polymeric compound may be dissolved in the aqueous environment.

As used herein, the term "contacting" is intended to mean placing into contact. The term "contacting an array with a solution" is intended to encompass direct contact between an array and solution, e.g., by dipping or misting, as well as depositing a solution on the surface of the substrate by condensation.

As used herein, the term "bound to the substrate via a cleavable linker" is intended to refer to an arrangement in which a polymeric compound is linked to a substrate via a cleavable bond. A cleavable bond may be cleaved using base (e.g., ammonia or trimethylamine), acid, fluoride or photons, for example.

As used herein, the term "a pre-defined combination of oligonucleotides" is intended to refer to a combination of oligonucleotides, where the combination was planned beforehand.

As used herein, a "mixture of oligonucleotides" refers to an aqueous solution that contains a plurality of different oligonucleotides dissolved therein. A mixture may comprise at least 50, at least 100, at least 500 at least 1,000, at least 5,000, at least 10,000 or at least 50,000 or more of oligonucleotides. A mixture of oligonucleotides may be made by synthesizing the oligonucleotides *in situ,* i.e., synthesizing the oligonucleotides in place in an array and then cleaving the oligonucleotides from the surface of the array after they have been synthesized. Examples of such methods are described in, e.g., Cleary et al. (Nature Methods 2004 1: 241-248) and LeProust et al. (Nucleic Acids Research 2010 38: 2522-2540). In this example, the oligonucleotides may be cleaved using base (e.g., ammonia or trimethylamine), acid, fluoride or photons, for example.

As used herein, the term "multiple sets", in the context of a composition comprising multiple sets of oligonucleotides, refers to multiple distinct populations of oligonucleotides, where a set of oligonucleotides may comprise at least 2, at least 5, at least 10, at least 50, or at least 100 or more (e.g., 3 to 50, e.g., 4 to 30) of oligonucleotides and the composition may contain at least 5, at least 10, at least 50, at least 100, at least 500, at least 1,000 or at least 5,000 or more sets of oligonucleotides.

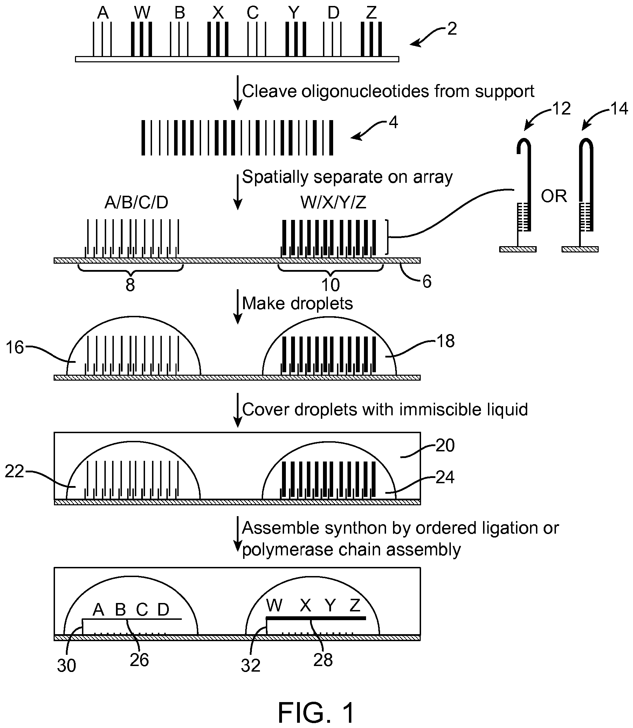

As used herein, the term "a set of oligonucleotides that can be assembled to produce a synthon" and grammatical equivalents thereof refers to a set of oligonucleotides that can be enzymatically assembled into a longer sequence, referred to herein as a "synthon", that contains sequences from each of the oligonucleotides in a defined order. As would be understood, the oligonucleotides of a set comprise: (i) a terminal indexer sequence and (ii) an assembly sequence, wherein the assembly sequences of each set of oligonucleotides can be assembled to produce a synthon. Typically, the terminal indexer sequences are removed from at least some of the oligonucleotides before the actual assembly takes place. For example, the assembly sequences of a set of oligonucleotides can be assembled: (i) after all but one of the terminal indexer sequences have been cleaved from the assembly sequences (in which case the synthon will be tethered to the array; see Fig. 1) or (ii) after all of the terminal indexer sequences have been cleaved from the assembly sequences (in which case the synthon will be in solution and not tethered to the array). Sequence assembly can be done using a variety of different methods, including, but not limited to polymerase chain assembly (Hughes, et al. Methods in Enzymology 2011 498:277-309 and Wu, et al. J. Biotechnol. 2006, 124:496-503) and ordered ligation. Gibson assembly (Gibson Methods in Enzymology 2011 498: 349-361) could be used in certain circumstances. In some embodiments, the oligonucleotides are digested with a restriction enzyme or using a light or chemical stimulus before the assembly.

As used herein, the term "polymerase chain assembly", refers to a protocol in which multiple overlapping oligonucleotides are combined and subjected to multiple rounds of primer extension (i.e., multiple successive cycles of primer extension, denaturation and renaturation in the presence of a polymerase and nucleotides) to extend the oligonucleotides using each other as a template, thereby producing a product molecule. In some cases, the final product molecule can be amplified using primers that bind to sites at the ends of the product molecule.

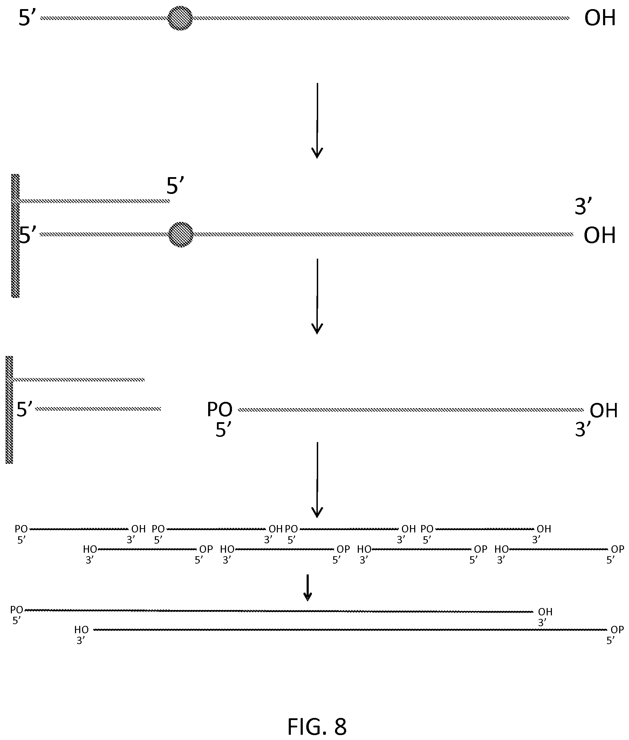

As used herein, the term "ordered ligation", refers to a protocol in which double-stranded fragments are ligated to one another to produce a synthon using DNA ligase, where the order of fragments in the synthon is dictated by the sequences of the overhangs that are ligated together. The term "ordered ligation" also refers to a protocol in which single-stranded oligonucleotides hybridize to one another in a defined order and can be ligated together to make a synthon (see, e.g., the bottom of Fig. 8).

In one example, a set of oligonucleotides that can be assembled to produce a synthon is a set of single stranded primers that have overlapping ends such that each of the primers can be extended using another primer as a template and can produce a synthon by polymerase chain assembly.

In another example, a set of oligonucleotides that can be assembled to produce a synthon is a set of oligonucleotides that, in their double-stranded form, are digestible by a Type IIs restriction endonuclease to produce fragments that that are ligatable to one another in a defined order, thereby producing the synthon.

In another example, a set of oligonucleotides that can be assembled to produce a synthon is a set of double stranded oligonucleotides that contain cleavable linkages that are staggered, such that cleavage of the double stranded oligonucleotides by an appropriate stimulus (e.g., a chemical agent or a light stimulus) results in fragments that are ligatable to one another in a defined order.

In another example, a set of oligonucleotides that can be assembled to produce a synthon is a set of single-stranded oligonucleotides that each contain a cleavable linkage, such that cleavage of the single-stranded oligonucleotides by an appropriate stimulus (e.g., a chemical agent or a light stimulus) results in oligonucleotides that can hybridize to one another in a defined order and be ligated to one another to produce a synthon.

As used herein, the term "terminal indexer sequence" refers to a unique sequence that occurs at or near the end of a population of oligonucleotides, wherein, the oligonucleotides within each set of oligonucleotides have the same indexer sequence and each set of oligonucleotides has a different indexer sequence. Indexer sequences are different from one another or their complements. For example, a first unique sequence has a different nucleotide sequence than a second unique sequence or its complement. Indexer sequences do not hybridize to each other, i.e., they have been designed so that they do not anneal to one another under stringent conditions. Such sequences, called "sequence tokens" in certain publications, are described in, e.g., US20070259357 and Brenner et al (Proc. Natl. Acad. Sci. 1992 89:5381-3). A terminal indexer sequence may be 8-50 bases in length, e.g., 10-35 bases in length. In some instances, a terminal indexer sequence may be up to 100 bases in length.

As used herein, the term "spatially-separating" in the context of spatially-separating different sets of oligonucleotides from one another, refers to separating different sets of oligonucleotides from one another such that the different sets of oligonucleotides are present at different locations on an array. Specifically, the oligonucleotides in a first set become associated with a first location on an array, the oligonucleotides in a second set become associated with a second location on the array, and the oligonucleotides in a third set become associated with a third location on the array, and so on.

As used herein, the term "single-stranded oligonucleotide" refers to an oligonucleotide molecule that is mostly single stranded. A single stranded oligonucleotide may have a short hairpin at one end (e.g., a hairpin with a stem of 8-30 base pairs).

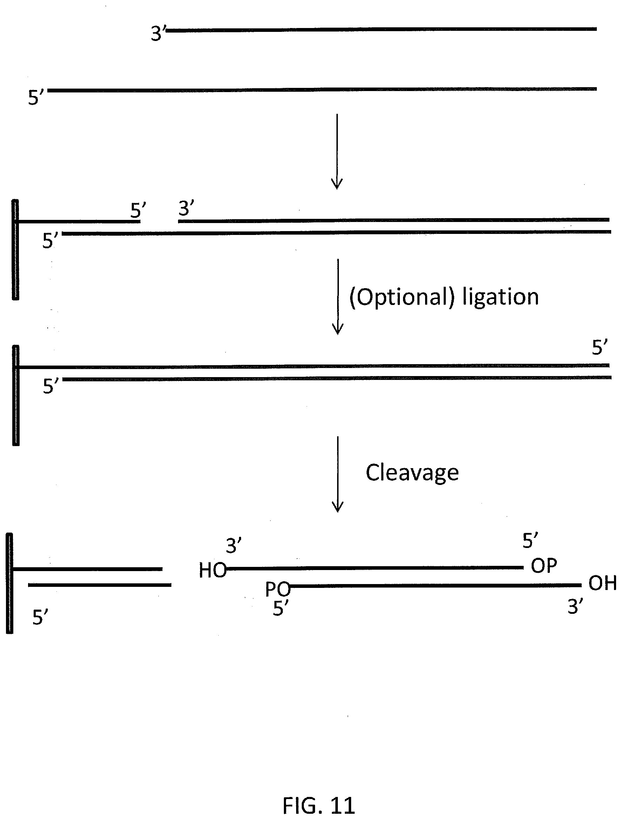

As used herein, the term "double stranded oligonucleotide" refers to an oligonucleotide molecule that is substantially double-stranded, e.g., contains a double stranded region of at least 50 base pairs. A double stranded oligonucleotide may be synthesized as a long hairpin. In some embodiments, a double-stranded oligonucleotide may be composed of single stranded oligonucleotides that are hybridized together, as shown in Figs. 11-14.

The term "synthon", as used herein, refers to a synthetic nucleic acid that has been assembled *in vitro* from several shorter nucleic acids.

Other definitions of terms may appear throughout the specification.

### DESCRIPTION OF EXEMPLARY EMBODIMENTS

Before the various embodiments are described, it is to be understood that the teachings of this disclosure are not limited to the particular embodiments described, and as such can, of course, vary. It is also to be understood that the terminology used herein is for the purpose of describing particular embodiments only, and is not intended to be limiting, since the scope of the present teachings will be limited only by the appended claims.

The section headings used herein are for organizational purposes only and are not to be construed as limiting the subject matter described in any way. While the present teachings are described in conjunction with various embodiments, it is not intended that the present teachings be limited to such embodiments. On the contrary, the present teachings encompass various alternatives, modifications, and equivalents, as will be appreciated by those of skill in the art.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present teachings, some exemplary methods and materials are now described.

The citation of any publication is for its disclosure prior to the filing date and should not be construed as an admission that the present claims are not entitled to antedate such publication by virtue of prior invention. Further, the dates of publication provided can be different from the actual publication dates which can be independently confirmed.

As will be apparent to those of skill in the art upon reading this disclosure, each of the individual embodiments described and illustrated herein has discrete components and features which can be readily separated from or combined with the features of any of the other several embodiments without departing from the scope or spirit of the present teachings. Any recited method can be carried out in the order of events recited or in any other order which is logically possible.

### Methods

Some features of a method are illustrated in Fig. 1. In certain embodiments the method may comprise (a) obtaining an aqueous mixture **4** of multiple sets of oligonucleotides. In this example, the first set of oligonucleotides is composed of oligonucleotides A, B, C and D and the second set of oligonucleotides is composed of oligonucleotide W, X, Y and Z, where, in this example, the oligonucleotides were synthesized *in situ* on the surface of the same substrate (e.g., using the methods of e.g., Cleary et al., Nature Methods 2004 1: 241-248, LeProust et al., Nucleic Acids Research 2010 38: 2522-2540) and then cleaved from the support. As illustrated, the oligonucleotides do not need to be synthesized in any particular order on substrate **2.** As noted above, each set of oligonucleotides can be assembled to produce a synthon, i.e., can be enzymatically assembled into a longer sequence that contains sequences from each of the oligonucleotides in a defined order. In some embodiments, the oligonucleotides, in their double-stranded form, are digestible by a Type IIs restriction enzyme or cleavable by a chemical or light to produce fragments that can be assembled by polymerase chain assembly or by ordered ligation. In some cases, e.g., in embodiments that rely on polymerase chain assembly, each oligonucleotide in a set may have a region of complementarity (e.g., at least 8, at least 12 or at least 15 nucleotides) to another oligonucleotide in the same set, or the complement thereof. In one example, a set of oligonucleotides that can be assembled to produce a synthon is a set of single stranded primers that have overlapping ends such that each of the primers, after cleavage, can be extended using another primer as a template and can produce a synthon by polymerase chain assembly.

As noted above, the oligonucleotides within each set each comprise a terminal indexer sequence and can be assembled to produce a synthon. In other words, the oligonucleotides within each set can be assembled to produce a synthon, the oligonucleotide within each set contain the same terminal indexer sequence, and the oligonucleotides in the different sets differ from one another by their indexer sequence. Next, the method comprises (b) hybridizing the oligonucleotide mixture to array **6,** thereby spatially-separating the different sets of oligonucleotides from one another. In these embodiments, the oligonucleotides of one set locate to one feature and the oligonucleotides of another set locate to another feature. As illustrated, oligonucleotides A, B, C and D locate to feature **8** and oligonucleotide W, X, Y and Z locate to feature **10.** As shown, the oligonucleotides can hybridize directly to oligonucleotides that are immobilized on the array. In other embodiments (not shown in Fig. 1) described in greater detail below, the oligonucleotides may hybridize to oligonucleotides that are immobilized on the array via an adaptor oligonucleotide. As shown, and as will be described in greater detail below, the hybridized oligonucleotides may be single stranded (e.g., **12**) and, in certain cases may comprise a 3' hairpin that can be extended to produce a double-stranded extension product. In other embodiments, the oligonucleotides may be double-stranded oligonucleotides (e.g., **14**).

Next, the method may further comprise: (c) contacting the array with a solution, thereby producing, for each feature bound by the oligonucleotides, a discrete droplet comprising one or more features. As shown, in Fig. 1, feature **8** is encapsulated in droplet **16** and feature **10** is encapsulated in feature **18**). In these embodiments, the solution contains all of the necessary reagents (which may include any one or more of, e.g., a polymerase, nucleotides, ligase, buffer and ATP) for assembly of the hybridized oligonucleotide into a synthon. For example, if a hairpin oligonucleotide is used, then the method may further comprise contacting the array with a solution comprising a polymerase and nucleotides (and, optionally, other reagents including, for example, ligase and ATP), thereby extending the hairpin and producing, for each feature bound by the oligonucleotides, a set of double-stranded extension products. If an adaptor oligonucleotide is used, the method may comprise contacting the array with a solution comprising a polymerase and nucleotides (and, optionally, ligase and ATP), thereby extending the adaptor and producing, for each feature bound by the oligonucleotides, a set of double-stranded extension product. As noted above, this method may be done in a variety of different ways and may be done by dipping the array into an aqueous solution containing all of the necessary reagents, where the difference in hydrophobicity between the features and non-feature areas confines the droplets of liquid to the features.

Next, the method may further comprise placing an immiscible liquid **20** over the droplets, thereby producing, for each feature bound by the oligonucleotides, a discrete reaction chamber (i.e., a droplet that is encapsulated on all sides, i.e., by the substrate in one side and by the immiscible liquid on the other). As shown in Fig. 1, addition of immiscible liquid 20 results in reaction chambers 22 and 24. The immiscible liquid can comprise a mineral oil such as Petroleum Special, an alkane such as heptadecane, a halogenated alkane such as bromohexadecane, carbonated oils, perfluorocarbons, partially fluorinated liquids, e.g. 3M's Novek™ HFE-7500, an alkylarene, a halogenated alkylarene, an ether, or an ester having a boiling temperature above 100° C, for example. The immiscible liquid should be insoluble or slightly soluble in water. In certain cases, the liquid may or may not contain added surfactants that have hydrophilic-lipophilic-balances (HLB) values equal to, less than or more than, e.g., 4,0, 5.0, 6,0, 7.0, 8,0, 9.0, or 10,0. Those who are skilled in the art can appreciate that the surfactant affinity difference (SAD) of an oil phase can be adjusted by using various surfactants with various HLB values such that a stable droplets can be prepared. For example, fluorinated carrier oils (surfactants with fluorinated tails) that reduce the solubility of non-fluorinated compounds could be used. In certain cases, silicone oils, mineral oils, carbon-chain oils, and all fluorinated polycarbon chains and their modifications, or small non-polar molecules (carbon-tetracholoride, ether, chloroform, etc.) could be used. In these embodiments, the droplets may be encapsulated in the immiscible liquid by gently pipetting the immiscible liquid on top of the array, flowing fluids through a flow cell or chamber that includes the array substrate, although other methods (e.g., dipping the array into the immiscible liquid) may also be used.

Next, the reaction chambers are maintained under conditions by which assembly of the oligonucleotides into a synthon occurs. This step of the method may involve maintaining the array under isothermal conditions (e.g., a temperature in the range of 30 °C and 75 °C (depending on the enzymes used). However in some cases, the array may be thermocycled. In the example shown in Fig. 1, the reaction results in synthon **26** (which comprises sequences from oligonucleotides A, B, C and D) and synthon **28** (which comprises sequences from oligonucleotides W, X, Y and Z). The oligonucleotides (or extended versions of the same) may be assembled into a synthon using polymerase chain assembly or ordered ligation, for example.

In polymerase chain assembly embodiments, multiple overlapping oligonucleotides are combined and subjected to multiple rounds of primer extension (i.e., multiple successive cycles of primer extension, denaturation and renaturation in the presence of a polymerase and nucleotides) to extend the oligonucleotides using each other as a template, thereby producing a product molecule. In embodiments that use polymerase chain assembly, the initial sets of oligonucleotides that can be assembled to produce a synthon may be sets of single stranded primers that have overlapping ends such that each of the primers can be extended using another primer as a template and can produce a synthon by polymerase chain assembly.

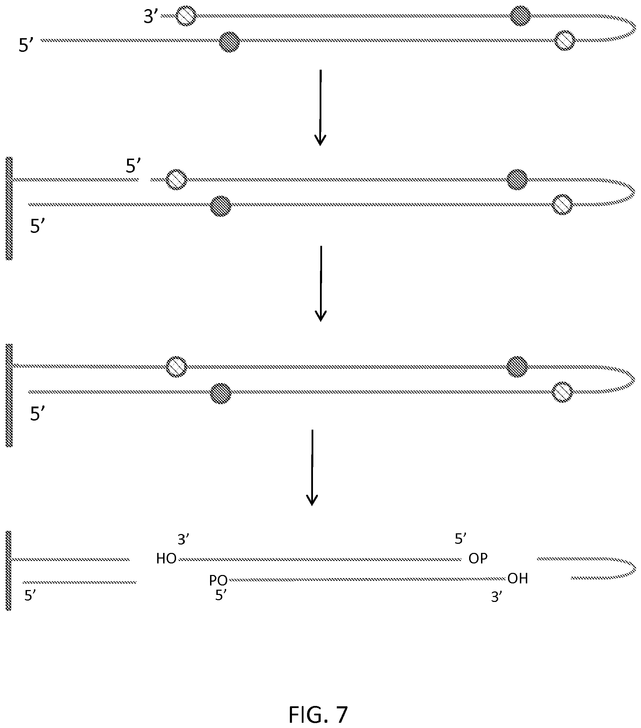

In ordered ligation methods, double-stranded fragments are ligated to one another to produce a synthon using DNA ligase, where the order of fragments in the synthon is dictated by the sequences of the overhangs that are ligated together. In these embodiments, the initial sets of oligonucleotides that can be assembled to produce a synthon can be sets of oligonucleotides that, in their double-stranded form, are digestible by a Type IIs restriction endonuclease to produce fragments that that are ligatable to one another in a defined order, thereby producing the synthon. In alternative embodiments, the initial sets of oligonucleotides that can be assembled to produce a synthon can be sets of double stranded oligonucleotides that contain staggered cleavable linkages, where, after cleavage of the linkages by an appropriate stimulus, fragments that are ligatable to one another are produced. This embodiment is illustrated in Fig. 7. In this embodiment, oligonucleotides containing chemically- or photo-cleavable linkers in the appropriate places are used. The 5' end contains the terminal indexer sequence that will bind to the array, and the circles represent two types of cleavable linkers. The oligonucleotides are hybridized to the array. In certain cases, an optional ligation step can be performed. In these embodiments, the 5'-end of the oligonucleotide on the microarray may be synthesized to contain the required terminal phosphate, or a kinase is used to phosphorylate it. After ligation, the oligo is covalently attached to the surface. If no ligation step is done, the cleavable linker at the 3'-end is not needed, and the original oligonucleotide can be directly synthesized to have the desired terminal 3'-OH. In either case, a wash is performed to remove unwanted oligos. The chemically cleavable linkers need to be cleaved under conditions such that the cleaved oligo maintains its spatial location on the surface without possible cross-contamination, for example, using gaseous ammonia or light.

Two different chemically cleavable linkers may be needed in some cases. Some linkers should leave behind a 3'-OH, while other linkers should leave behind a 5' phosphate. A suitable phosphoramidite that can be used for terminal 5'-phosphate formation is shown below. This and other suitable linkers that leave behind a 5'-terminal phosphate can be obtained from commercial sources.

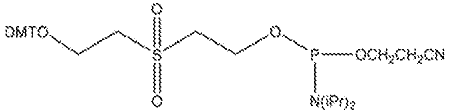

For the 3'-hydroxyl terminated end of the oligonucleotide, suitable phosphoramidite monomers are known and include the Unylinker®-related phosphoramidite (US20040152905) and thymidine-succinyl hexamide CED phosphoramidite (Chemgenes). Using the thymidine-succinyl hexamide CED phosphoramidite linker, the will result in an oligonucleotide terminated by a T residue at the 3'-end. Alternative linkers could be constructed so that the oligonucleotide is terminated by any desired base.

After chemical cleavage with gaseous ammonia, the ligation enzyme required to assemble the sticky-ended oligonucleotides may be added to the droplet that will cover each feature.

Photochemically cleavable linkers can also be used. Since the features have not yet been cleaved, they can be covered with individual droplets containing the ligase enzyme and buffer without having to be concerned about cross contamination (generated by, for example, dipping the slide in a bulk reservoir of reagents followed by careful removal to leave 'reagent droplets' behind on the features but not on the surface between features). After the individual features are covered with the solution of aqueous buffer and enzyme (and optionally a non-miscible fluid to prevent evaporation), the slide is exposed to the appropriate wavelength of light in order to cleave the oligos. Subsequent ligation of the sticky ended oligonucleotides can proceed in the same droplet.

A commercially available (Glen Research) photocleavable linker phosphoramidite that leaves behind the desired phosphate on the 5'-end is shown below.

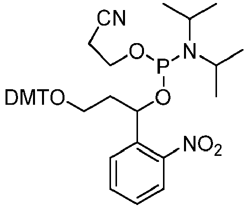

A suitable photocleavable linker photocleavable linkers that leaves behind a 3'-OH is shown below.

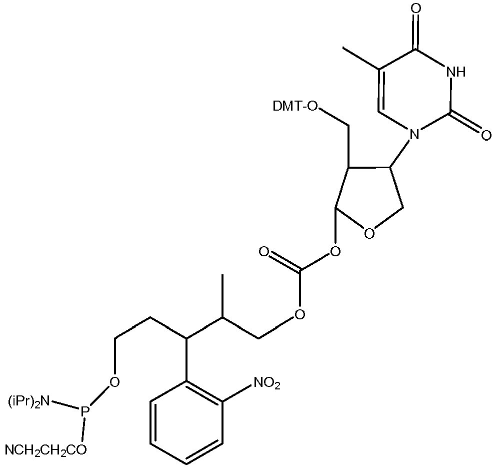

With such a linker the last residue may be incorporated into the linker structure itself, and if desired, alternative photocleavable linkers incorporating any base as the last residue could be synthesized.

How a synthon may be assembled using oligonucleotides that contain photocleavable or chemically cleavable linkers is described in further detail in the Examples section of this disclosure and in Figs. 7 and 8.

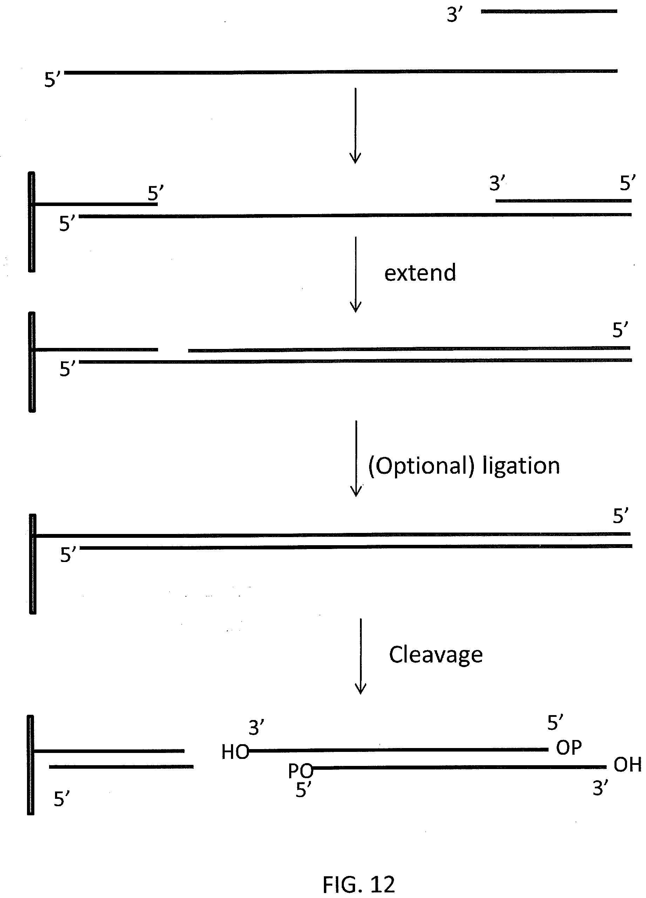

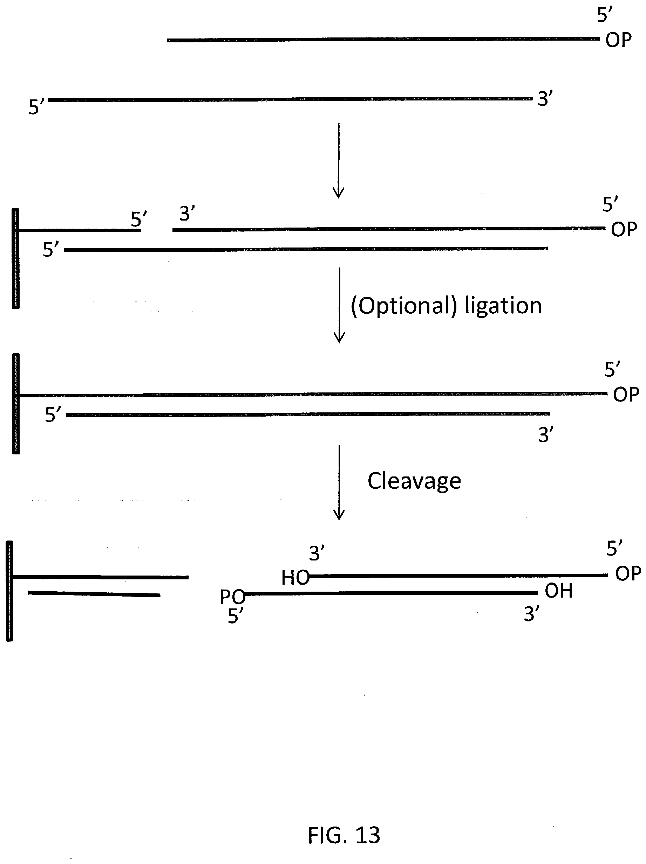

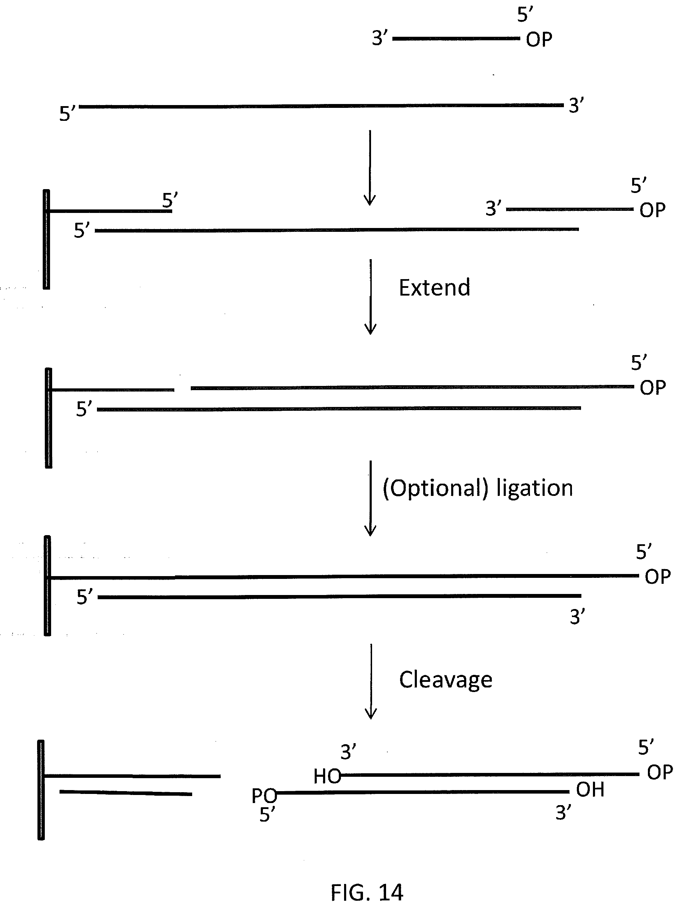

Alternative methods for producing assemblable cleavage products are shown in Figs. 11-14. In these embodiments, single stranded oligonucleotides that do not contain a hairpin are hybridized to one another to make a duplex, and one of the oligonucleotides of each of the duplexes contains a terminal indexer sequence that hybridizes (directly or indirectly to via an adaptor) to oligonucleotides that are immobilized on the array. The immobilized duplexes are then cleaved (using, e.g., a Type IIS restriction endonucleases, or a chemical or light stimulus, as described above) and are assembled with one another using any suitable method. As shown in Figs. 11 and 12, each duplex may be cleaved at two sites (one site being proximal to the array and the other site being distal to the array), to produce a fragment that contains appropriate overhanging ends. In other embodiments (as shown in Figs. 13 and 14), the duplex may already contain one of the overhangs prior to cleavage. In these embodiments, the duplex may be cleaved at a single site (which is proximal to the array), to produce a fragment that contains appropriate overhanging ends.

As shown in Fig. 1, the synthons, after they are made, may be immobilized on the array, e.g., by linkers **30** and **32.** In other embodiments, the synthons may be in solution in the reaction chamber (i.e., not immobilized to the array). This may be done by, e.g., digesting the synthon with a restriction enzyme or by cleaving a linker that immobilizes the synthon to the array. In some embodiments, the assembly may be done using a set of cleavage products that are all in the solution phase, resulting in a synthon that is not immobilized to the array. In these embodiments, the method may involve, spatially separating a set of oligonucleotides from other sets of oligonucleotides, cleaving the oligonucleotides (e.g., using a chemical or light stimulus if the oligonucleotides are single stranded, or a chemical or light stimulus, or a restriction enzyme if the oligonucleotides are double stranded) and then assembling the products, which are now in the solution phase, into a synthon. In certain cases (particularly if the synthons are immobilized on the array), the immiscible fluid and droplets may be removed, and the surface of the array may be washed prior to release of the synthons from the array. The synthons can then be collected in the aqueous phase and, in certain cases, amplified using primers (e.g., universal primers) that bind to sites at the ends of the synthons. The method may be used to make at least 10, at least 50, at least 100, at least 500, at least 1,000, at least 5,000, at least 10,000 or at least 50,000 synthons in parallel. In other embodiments, the synthons may by amplified *in situ,* i.e., while they are attached to the surface of the array (e.g., by linkers **30** and **32**), using primers that are in the solution phase.

Type IIS restriction endonucleases are restriction enzymes that have a recognition site that is offset from the cut site. There are numerous endonucleases that are useful for the gene assembly method described here. The following is an incomplete list of Type II enzymes that may be used: Acc36I, AceIII, AcuI, AlfI, AloI, Alw26I, AlwXI, BaeI, Bbr7I, BbsI, Bbv16II, BbvI, BbvII, Bce83I, BceAI, Bcefl, BcgI, Bco116I, Bco5I, BcoKI, BfuAI, Bli736I, Bme585I, BpiI, BplI, BpmI, BpuAI, BpuEI, BpuJI, BpuSI, BsaI, BsaXI, Bsc91I, BscAI, Bse3DI, BseGI, BseKI, BseMI, BseMII, BseRI, BseXI, BseZI, BsgI, BslFI, BsmAI, BsmBI, BsmFI, Bso31I, BsoMAI, Bsp24I, Bsp423I, BspBS31I, BspCNI, BspD6I, BspIS4I, BspKT5I, BspLU11III, BspMI, BspST5I, BspTNI, BspTS514I, BsrDI, Bst12I, Bst19I, Bst6I, Bst71I, BstBS32I, BstF5I, BstFZ438I, BstGZ53I, BstMAI, BstOZ616I, BstPZ418I, BstTS5I, BstV1I, BstV2I, Bsu6I, BtgZI, BtsI, BveI, CjeI, CjePI, CspCI, CstMI, Eam1104I, Earl, EciI, Eco31I, Eco57I, Eco57MI, EcoA4I, EcoO44I, Esp3I, Fall, FaqI, FauI, FokI, GsuI, HaeIV, HgaI, Hin4I, Ksp632I, LweI, MmeI, PhaI, PpiI, PsrI, RleAI, SapI, SfaNI, SmuI, Sth132I, StsI, TaqII, TspDTI, TspGWI, Tth111II, and VpaK32I. One attribute these enzymes all have in common is that they leave one end with an overhang that has no specific recognition sequence. They have varying degrees of utility due to the number of bases in the overhang, the distance between the recognition site, and efficiency with which they cleave the substrate. Either 5' or 3' overhangs are created by different Type IIS enzymes. Either type of overhang can be utilized for this method, as can combinations or mixtures of enzymes, either for the purpose of enhancing cleavage efficiency or overhang diversity and specificity.

The synthon itself can be of any sequence and, in certain cases, may encode a sequence of amino acids, i.e., may be a coding sequence. In other embodiments, the synthon can be a regulatory sequence such as a promoter or enhancer. In particular cases, the synthon may encode a regulatory RNA. In certain cases a synthon may have a biological or structural function.

In particular cases, a synthon may be cloned into an expression vector designed for expression of the synthon. In these embodiments, the expression vector may contain a promoter, terminator and other necessary regulatory elements to effect transcription and in certain cases translation of the synthon, either as a single protein, or as a fusion with another protein. In these embodiments, the method may further comprises transferring the expression vector into a cell to produce the expression product (e.g., a protein) encoded by the synthon. This embodiment of the method may comprise screening the expression product for an activity.

Also provided is a composition comprising multiple sets of oligonucleotides, wherein the oligonucleotides within each set comprise a terminal indexer sequence and can be assembled to produce a synthon. In some compositions, the oligonucleotides, in their double-stranded form, are digestible by a Type IIs restriction enzyme to produce fragments that can be assembled by ordered ligation or polymerase chain assembly. In particular cases, the composition may comprise: a first set of synthetic oligonucleotides of formula A-X, wherein A is a terminal indexer sequence that is common to all of the oligonucleotides in the first set and X is different in the oligonucleotides in the first set; wherein the oligonucleotides in the first set, in their double-stranded form, are digestible by a Type IIs restriction enzyme to produce fragments that can be assembled with one another in a defined order; and a second set of synthetic oligonucleotides of formula B-Y, wherein B is common to all of the oligonucleotides in the second set and is different to A, and wherein Y is different in the oligonucleotides in the second set; wherein the oligonucleotides in the second set, in their double-stranded form, are digestible by a Type IIs restriction enzyme to produce fragments that can be assembled with one another in a defined order.

### EXAMPLES

The following examples describe two related methods for the assembly of -1,500 base-pair constructs starting from 200 nucleotide oligonucleotides (200mers) that are synthesized on a microarray surface and then cleaved off the substrate thereby generating a complex mixture of oligonucleotides. The 1.5 kbp construct is assembled in defined features on a separate microarray. In this format, 32,000 1.5 kbp constructs can be simultaneously assembled on one microarray slide. This corresponds to the assembly of 48 million base-pairs. As would be apparent, this description is exemplary. The methods are not limited to 1.5 kbp constructs and the methods can be extended to construct genes of any length or sets of genes of different lengths.

### Example One

### Stating materials

Standard Microarray slide: Each feature on a microarray slide, for example ∼4,000 features each comprising a ∼50mer probe having a 5' 25mer unique sequence to address and capture each of the oligonucleotides from the oligonucleotide library that are required for the assembly and 3' 25mer stilt region. In this example, the 3' end of the oligonucleotide probes are attached to the surface of the microarray. This example assumes that there are ∼4 x 10⁻¹⁷ moles of probe per feature.

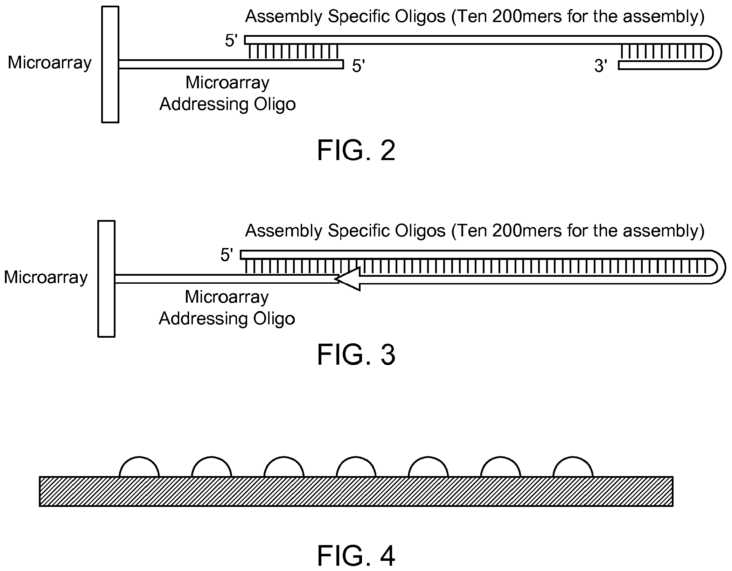

Oligonucleotide library synthesis (OLS): A 244,000 feature slide of ∼200mer oligonucleotides, each having a working payload of 150 nucleotides, is synthesized for a batch of 4,000 1.5 kbp assemblies (4,000 assemblies x 10 oligonucleotides per assembly x 6 redundant features per oligonucleotide = 240,000 total features), where all oligonucleotide sequences for a given assembly have a common 5' 25mer sequence complementary to a microarray probe feature (of a separate microarray used for oligonucleotide assembly) and a 3' hairpin sequence to serve as a polymerase primer site required to generate the second strand of the DNA. An example of such a hairpin oligonucleotide is schematically illustrated in Fig. 2. For OLS, one can assume 10% full-length yield, which will provide ∼2.5 x 10⁻¹⁷ moles of each oligonucleotide. Each oligonucleotide will also contain two distinct Type IIs restriction endonuclease sites, one near its 5' end and the other near the 3' end of resulting dsDNA that when cleaved will drive the assembly of the ∼150 base-pair fragment using DNA ligase. Here, the number 10 oligonucleotides per assembly is exemplary, the number needed could be as small as two and as large as hundreds or more.

A variation of this example is that both strands of the dsDNA fragment are provided by the OLS by synthesizing a 200mer having a hair-pin structure with a 5' 25mer overhang sequence that is complementary to the microarray probe feature. While this method eliminates the need to fill in the oligonucleotide with a DNA polymerase and also enables an error reduction step using MutS or a T7 endonuclease during or after synthesis, it limits the size of each fragment that is ligated into the larger construct to no greater than 100 base-pairs, using current chemistries. This will either limit the length of the final assembled product or increase the number of fragments to be ligated together in order to assemble the longer length products.

### On Microarray DNA Polymerization

The oligonucleotide library mixture is added to a standard addressing microarray in a hybridization chamber and the mix is hybridized to co-locate all oligonucleotides required for a given assembly to each defined feature on the microarray. After washing, the DNA polymerase (e.g., a 5' to 3' exo-minus polymerase), dNTPs, DNA ligase and ATP are then added to the hybridization chamber to generate and covalently anchor the double stranded DNA corresponding to all fragments for a specific assembly defined for each microarray feature. In some cases, e.g., if the full hairpin oligonucleotide library is hybridized to the microarray, the DNA polymerization step can be eliminated, but the synthesis still benefits from a ligation step to robustly anchor the duplex to the feature probes. An example of such an extension product is schematically illustrated in Fig. 3.

The reaction mixture is incubated and then washed to remove unbound extended oligonucleotides. In certain cases the washing step may benefit from the addition of some additives to increase the hydrophobic-hydrophilic boundary between the features and the interstitial substrate regions.

### On Microarray Assembly

The microarray slide is then exposed to or "dipped" into solution comprising a buffer, Type II restriction endonuclease, DNA ligase and ATP. This exposure to buffered reagents can also be accomplished by flooding and purging substrates within an enclosed chamber, or dipping the substrate into a buffered enzyme solution. Because of the hydrophobic-hydrophilic boundary between the microarray features and the interstitial substrate, the aqueous solution will form small droplets over the hydrophilic features containing the DNA thereby isolating each assembly reaction defined by the microarray feature. The chambers that are created are schematically illustrated in Fig. 4.

The double stranded DNA fragments that are now attached to microarray probes are designed to have standard Type IIs restriction enzyme sites (e.g. Bsa1) at both ends which when cleaved, create 5'-terminal overhangs ("sticky ends"), each overhang having a short unique sequence (4 - 6 nucleotides) within each droplet, which will drive the order in which the various fragments are ligated together. The IIs subclass of restriction enzymes cut nucleic acids at an offset displaced from the recognition site. This property allows the oligonucleotide designer to design fragment sequences with unique overhang sequences using a common restriction enzyme for all fragments. These distinct overhang sequences enable the ordered self-assembly of long constructs such that each component assembles in the proper order and orientation as determined by the overhanging sequence of each fragment. It is estimated that the DNA concentration for each of the 10 DNA fragments within the hemispherical droplet is >50nM, which is sufficient to drive the hybridization of the complementary "sticky ends" for ligation. Note that if the 5'-terminal fragment contains a second Type IIS restriction enzyme cut site at its 5'-end (e.g. FokI), the assembled DNA construct will remain attached to the microarray surface allowing additional post-assembly washing steps, if necessary. The assembled construct can then be liberated from the substrate by cleaving with the second restriction endonuclease, or it could be left attached for subsequent amplification while still attached to the surface. Alternatively, a chemically cleavable linker can be used to liberate the constructs from the substrate.. The released assembled constructs are then amplified using PCR either individually or in sets of assemblies as defined by the PCR primer binding sites within the 5' and 3' ends of the assembled constructs. In this way multiple gene sets, each comprising a distinct specific pathway or mechanism, can be isolated independently from the same complex pool of constructs.

If necessary, a sequence error reduction step can be performed with an endonuclease that recognizes and cleaves at mismatches, such as T7 endonuclease, at the oligonucleotide synthesis step or after assembly on the microarray, and prior to release, within the isolated feature droplets. For the latter process, the microarray may be heated in a controlled-humidity chamber (or with an added reagent that minimizes evaporation) to form heteroduplexes within the isolated droplets and then dipped into a solution containing T7 endonuclease to destroy all molecules containing mismatches and bulges. The final error-reduced mixtures are then amplified and isolated as described above.

To facilitate the effective isolation of the reaction volumes of each oligonucleotide feature, the droplets may be immersed in oil, or another fluid immiscible with aqueous solutions required for enzymatic activity. This oil or isolation fluid enables the reaction to continue for whatever time necessary for the assembly of the construct to reach completion for the bulk of the molecules within each droplet. Alternatively, the immiscible solution may be added by vaporizing a volatile isolation fluid, such as a short-chain fluorinated hydrocarbon, and allowing it to condense on the surface of the substrate and droplets there upon. Once a non-aqueous boundary layer covers the aqueous droplets additional non-aqueous fluids can be flowed into the chamber to improve the isolation of the droplets. Once in place, this isolation fluid enables substantial changes in temperature of the droplets, without mixing of droplets or droplet evaporation, to optimize reaction rates.

To control the reaction rates and the duration of each reaction, the humidity, in the absence of isolation fluid, and the temperature of the chamber housing the slide or wafer are controlled. One challenge of the assay is control of the relative reaction rate of the restriction enzymes used to liberate the fragments and the flow of reagents across the substrate to produce the isolated droplets. If the enzyme were to act instantly, many of the fragments may be washed away before droplet isolation can be achieved. This enzymatic reaction can be controlled by temperature and salt. Restriction enzymes have substantial temperature dependences with severely reduced enzymatic activity at temperatures below 10° C. (see, e.g., Fritz et al, Eur. J. BioChem, 123, 141-152 (1982)). Thus, by cooling the substrate and solution to a few degrees above freezing the activity of the enzyme can be effectively shut down. Subsequently, once the droplets are properly isolated, e.g. by carrier fluid, the substrate can be warmed to a temperature that optimizes the enzymatic activity. Thermophillic restriction enzymes, including *TaqII,* have activity as high as 65° C. If natural thermophillic enzymes do not provide sufficient efficiency, "hot-start", or, perhaps more appropriately, "warm-start" endonucleases can be created to delay the fragment cleavage until the starting temperature is achieved. Hot-start polymerases are generated by finding an antibody that binds to the active site of the polymerase (see, e.g., BioTechniques, 16(6), 1134-1137 (1994)). In this embodiment, the droplets are formed by immersion in a solution at a temperature below the "start" temperature of the nuclease, then the substrate is heated (either under precise humidity control or while protected by a biphasic surface emulsion, e.g. water-in-oil emulsion) to a temperature sufficient to remove the protective group or antibody, typically 25° C to 50° C and even as high as 70° C for a few endonucleases, then the enzymatic endonuclease reaction proceeds at an optimized temperature, as are any consequent polymerase and ligation reactions, at their respective optimized temperatures. More generally, the use of different enzymes with different optimal operating temperatures, can be used to stage the sequence of reactions, where each sequential reaction occurs at a higher temperature than the preceding reactions.

An alternate mechanism for controlling the enzymatic cleavage reaction is to deprive the solution of the divalent cations (usually magnesium) needed for enzymatic activity. It should be possible to add these divalent ions by means of the introduction of a salt in the form of an ionic carrier fluid, immiscible with the aqueous solution of the droplets. If the requisite salt is dissolved first in the isolation fluid, then as the carrier fluid reaches equilibrium with the aqueous solution after droplet encapsulation, it transfers some of its ions to the enzyme-loaded droplets.

### Example Two

### Starting Materials

Standard Microarray slide: Each feature on a microarray slide, for example ∼4,000 features, each comprises a ∼50mer probe having a 5' 25mer unique sequence to address and capture each of the AOM oligonucleotide (see below) that are required for the assembly and 3' 25mer stilt region. This example assumes that there are ∼4 x 10⁻¹⁷ moles of probe per feature.

Common Stock of 50mer Adaptor Oligonucleotide Mixture (AOM): The AOM oligos are synthesized by standard 384-well synthesis (IDT) and mixed into a mixture where the 5'-25mer sequence of each AO is complementary to one of the ∼4,000 standard microarray addressing sequences having a unique 3'-25mer sequence partner that is also unique among the ∼4,000 oligonucleotide set which will serve as a capture and DNA polymerase priming site for all OLS oligonucleotides required for each defined assembly (see below).

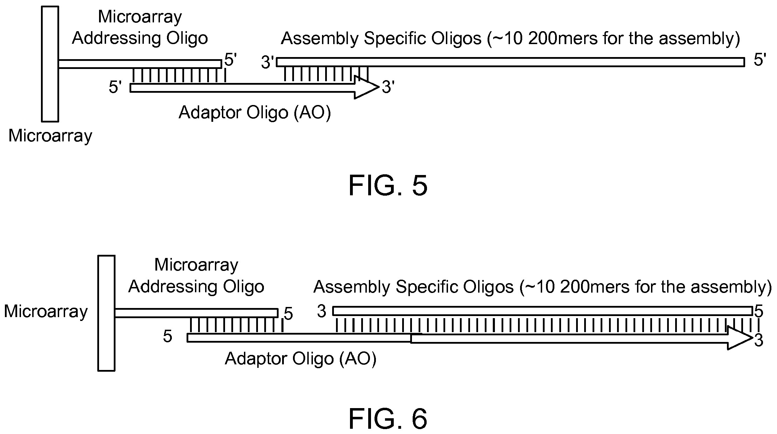

Oligonucleotide library synthesis (OLS): A 244K ∼200mer OLS having 150 nucleotides of useful sequence is synthesized for a batch of 4,000 1.5Kbp assemblies (4,000 assemblies x 10 oligonucleotides per assembly x 6 features per oligonucleotide = 240,000) where all oligonucleotides for a given assembly have a common 5' 25mer sequence complementary to a microarray probe feature. For OLS one can assume 10% full-length yield which will provide ∼2.5 x 10⁻¹⁷ moles of each oligonucleotide. Each oligonucleotide will also contain a Type IIs restriction endonuclease site at both the 5' and 3' ends of resulting dsDNA that when cleaved will drive the assembly of the ∼150 base-pair fragment using DNA ligase. Fig. 5 schematically illustrates an OLS oligonucleotide that is anchored to the microarray via an adaptor oligonucleotide.

### On Microarray DNA Polymerization

The OLS/AOM mixture is pre-hybridized to the OLS. This OLS/AOM mixture is then added to a standard microarray hybridization chamber and hybridized to co-locate all oligonucleotides required for a given assembly to each defined feature on the microarray. After washing, the DNA polymerase (e.g., a 5' to 3' exo-minus polymerase), dNTPs, DNA ligase and ATP are then added to the hybridization chamber to generate and covalently anchor the double stranded DNA corresponding to all fragments for a specific assembly defined for each microarray feature. Note that if the full hairpin OLS (see above) is hybridized to the microarray, the DNA polymerization step can be eliminated. Fig. 6 schematically illustrates a double-stranded OLS oligonucleotide that is hybridized by its terminal indexer sequence to a microarray.

The reaction mixture is incubated and then washed to remove unbound extended probe. The washing step may require addition of some additives to increase the hydrophobic-hydrophilic boundary between the features and the interstitial regions.

### On Microarray Assembly

The microarray slide is then "dipped" into solution comprising a buffer, Type II restriction endonuclease, DNA ligase and ATP. Because of the hydrophobic-hydrophilic boundary between the microarray features and the interstitial substrate, the aqueous solution will form small droplets over the hydrophilic features containing the DNA thereby isolating each assembly reaction defined by the microarray feature, as described above.

A more efficient embodiment than the "dipping" described above involves exposing the microarray to the enzyme within a flow cell, where reagents are sequentially flowed over the surface of the substrate in a precisely controlled process. This process is far more efficient in terms of the volumes of reagents needed.

The dsDNA fragments that are now attached to microarray probes have standard Type IIs restriction sites (e.g. Bsa1 sites) at both ends, which when cleaved, create 5'-terminal overhangs ("sticky ends") having distinct sequences, which will drive the order in which the various fragments are ligated together. The RS subclass of restriction enzymes cut nucleic acids at an offset displaced from the recognition site. This property allows the oligonucleotide designer to design fragment sequences with unique overhang sequences using a common restriction enzyme for all fragments. These distinct overhang sequences enable the ordered self-assembly of long constructs such that each component assembles in the proper order and orientation. It is estimated that the DNA concentration for each of the 10 DNA fragments within the hemispherical droplet is >50nM which is sufficient to drive the hybridization of the complementary "sticky ends" for ligation. Note that if the 5'-terminal fragment contains a second Type IIs restriction site at its 5'-end (e.g. FokI), the assembled DNA construct will remain attached to the microarray surface allowing additional post-assembly washing steps, if necessary. The assembled construct can subsequently be liberated by cleaving with the second restriction endonuclease. Alternatively, the assembled construct, lacking a second cleavage site, can be amplified in situ while it is attached to the array, using primers that are in the solution phase. The assembled constructs can be amplified using PCR either individually or in sets of assemblies as defined by the PCR primer binding sites within the 5' and 3' ends of the assembled constructs.

If necessary, an error reduction step can be performed with T7 endonuclease at the OLS step (above) or after assembly on the microarray, and prior to release, within the isolated feature droplets. T7 endonuclease is known to cut preferentially at bulges created by mismatches in the presence of a Manganese enriched buffer. For this error reduction process, the microarray is heated in a humidity chamber to form heteroduplexes within the isolated droplets and then exposed to a solution containing T7 endonuclease to destroy all molecules containing mismatches and bulges. The final error-reduced mixtures are subsequently amplified as described above.

### Example Three

This example, illustrated in Fig. 8, shows an approach that uses single-stranded oligonucleotides. In this example, use of cleavable linkers enables a single stranded assembly technique. A library of single stranded oligonucleotides containing a single cleavable linker, e.g., a photocleavable linker is prepared. The 5'end contains the terminal indexer that will bind to the array. The oligonucleotides are bound to the microarray via the terminal indexer. Because there is no need to synthesize a double stranded hairpin as in other approaches, the terminal indexer may be longer, allowing a more stringent wash without the need for ligation.

After the hybridization and washing, the ligation buffer system required for the final assembly is deposited on the individual features by dipping or other means. The slide is exposed to UV light of an appropriate wavelength (e.g. ∼340-370nm in the case of the previously illustrated linker-modifications can be made to change the wavelength), and the oligonucleotide is cleaved from the surface, leaving a 5'-phosphate on the cleaved oligonucleotide.

The single stranded oligonucleotides that shared the terminal indexer can then be assembled together into a larger construct in the same droplet without the further addition of reagents. As shown, one assembly method would be ligation of overlapping oligonucleotides without any gaps present between the oligonucleotides.

### EXEMPLARY EMBODIMENTS

Provided herein is a method comprising: (a) obtaining a mixture of multiple sets of oligonucleotides, wherein the oligonucleotides within each set each comprise a terminal indexer sequence and can be assembled to produce a synthon; and (b) hybridizing the oligonucleotide mixture to an array, thereby spatially-separating the different sets of oligonucleotides from one another. In any embodiment, oligonucleotides of each set can be assembled by polymerase chain assembly or ordered ligation.

In any embodiment, the mixture may comprise at least 50, at least 100, at least 500 at least 1,000, at least 5,000, at least 10,000 or at least 50,000 or more of the oligonucleotides.

In any embodiment, the mixture may comprise at least 5, at least 10, at least 50, at least 100, at least 500, at least 1,000 or at least 5,000 or more sets of the oligonucleotides.

In any embodiment, each set may comprise at least 5, at least 10, at least 50, or at least 100 or more (e.g., 3 to 50, e.g., 4 to 30) of the oligonucleotides.

In any embodiment, the terminal indexer sequence may be in the range of 10-50 nucleotides in length.

In any embodiment, the oligonucleotides may be single-stranded oligonucleotides.

In any embodiment, the oligonucleotides may involve degenerate bases, including N, Y, R, S, W, K, M, B, D, H and V, as defined by the IUPAC nucleotide code.

In any embodiment, the oligonucleotides may be double-stranded oligonucleotides.

In any embodiment, the oligonucleotides may be single stranded and comprise a 3' hairpin that can be extended to produce a double-stranded extension product.

In any embodiment, the method may further comprise contacting the array with a solution comprising a polymerase and nucleotides, thereby extending the hairpin and producing, for each feature bound by the oligonucleotides, a set of double-stranded extension products.

In any embodiment, the oligonucleotides may hybridize directly to oligonucleotides that are immobilized on the array.

Alternatively, the oligonucleotides may hybridize to oligonucleotides that are immobilized on the array via an adaptor.

In any embodiment, the method may comprise contacting the array with a solution comprising a polymerase and nucleotides (and, optionally, ligase and ATP), thereby extending the adaptor and producing, for each feature bound by the oligonucleotides, a set of double-stranded extension product.

In any embodiment, the method may further comprise: (c) contacting the array with a solution, thereby producing, for each feature bound by the oligonucleotides, a discrete droplet comprising one or more features.

In this embodiment, the method may further comprise placing an immiscible liquid over the droplets, thereby producing, for each feature bound by the oligonucleotides, a discrete reaction chamber defined by a droplet.

In this embodiment, the method may further comprise incubating the array under conditions by which a synthon is assembled in each of the reaction chambers.

In this embodiment, the droplets may comprise double-stranded oligonucleotides or double-stranded extension products, and the solution comprises a Type IIs restriction endonuclease, a DNA ligase and ATP.

In this embodiment, the products of digestion of the double-stranded oligonucleotides or double-stranded extension products by the Type IIs restriction endonuclease may be ligated to one another in a defined order by the DNA ligase in the discrete reaction chambers, thereby producing a synthon.

In some embodiments, the oligonucleotides are double-stranded oligonucleotides that comprise staggered photocleavable or chemically cleavable linkages, and wherein the method comprises cleaving the staggered cleavable linkages using light or a chemical treatment to produce fragments that are ligatable to one another in order.

In some embodiments, the droplets comprise double-stranded oligonucleotides or double-stranded extension products and the solution comprises a Type IIs restriction endonuclease, a DNA ligase, ATP, an exonuclease, a polymerase and nucleotides.

In some embodiments the oligonucleotides are single-stranded oligonucleotides and the method comprises: cleaving the terminal indexer sequence from the oligonucleotides to release the assembly sequences from the oligonucleotides; and assembling the synthon from the assembly sequences by polymerase chain assembly or by ligation.

In some embodiments, the cleaving may comprise cleaving a photocleavable linkage.

In any embodiment, the method may further comprise separating the synthons from the array. This embodiment may comprise cleaving the synthons from the array using a restriction enzyme.

In any embodiment, the method may further comprise amplifying the synthons by PCR, using primers that hybridize to the ends of the synthons.

In any embodiment, the ligation products in the discrete droplets may be coding sequences.

In some embodiments, all except for one of the double-stranded extension products have recognition sites for the same Type IIs restriction enzyme at both ends.

In any embodiment, the synthon may be at least 500 bp, at least 1 kb or at least 2kb bp in length.

In any embodiment, heteroduplexes may be removed after step (c) using an endonuclease that recognizes and cleaves at a mismatch, such as T7 endonuclease. In any embodiment, the fragments produced by digestion by the Type IIs restriction enzyme may be in the range of 50bp to 200 bp in length.

In any embodiment, the different sets are spatially separated on an array of oligonucleotides that are complementary to the indexer sequences.

In any embodiment, each droplet may comprise a Type IIs restriction endonuclease, a ligase, and a double-stranded ligation product.

In any embodiment, the array may comprise at least 100 different droplets, wherein each of the droplets comprises a different double-stranded ligation product.

Also provided is a composition comprising multiple sets of oligonucleotides, wherein the oligonucleotides within each set comprise a terminal indexer sequence and can be assembled to produce a synthon.

In some compositions, the oligonucleotides, in their double-stranded form, are digestible by a Type IIs restriction enzyme to produce fragments that can be assembled by polymerase chain assembly or ordered ligation.

In any embodiment, the mixture may comprise- at least 50, at least 100, at least 500 at least 1,000, at least 5,000, at least 10,000 or at least 50,000 or more of the oligonucleotides.

In any embodiment, the mixture may comprise at least 5, at least 10, at least 50, at least 100, at least 500, at least 1,000 or at least 5,000 or more sets of the oligonucleotides.

In any embodiment, each set may comprise at least 5, at least 10, at least 50, or at least 100 or more (e.g., 3 to 50, e.g., 4 to 30) of the oligonucleotides.

In any embodiment, the terminal indexer sequence may be in the range of 10-50 nucleotides in length.

In any embodiment, the composition may comprise: a first set of synthetic oligonucleotides of formula A-X, wherein A is an terminal indexer sequence that is common to all of the oligonucleotides in the first set and X is different in the oligonucleotides in the first set; wherein the oligonucleotides in the first set, in their double-stranded form, are cleavable (either using a Type IIs restriction enzyme or a chemical/light stimulus), to produce fragments that can be assembled (e.g., ligated to one another) in a defined order; and a second set of synthetic oligonucleotides of formula B-Y, wherein B is common to all of the oligonucleotides in the second set and is different to A, and wherein Y is different in the oligonucleotides in the second set; wherein the oligonucleotides in the second set, in their double-stranded form, are cleavable (either using a Type IIs restriction enzyme or a chemical/light stimulus), to produce fragments that can be assembled (e.g., ligated to one another) in a defined order.

In some embodiments a composition may comprise multiple sets of oligonucleotides, wherein the oligonucleotides within each set comprise : (i) a terminal indexer sequence and (ii) an assembly sequence, wherein the terminal indexer sequence and the assembly sequence are separated by a a photocleavable or chemically cleavable linker and the assembly sequences of each set of oligonucleotides can be assembled to produce a synthon.

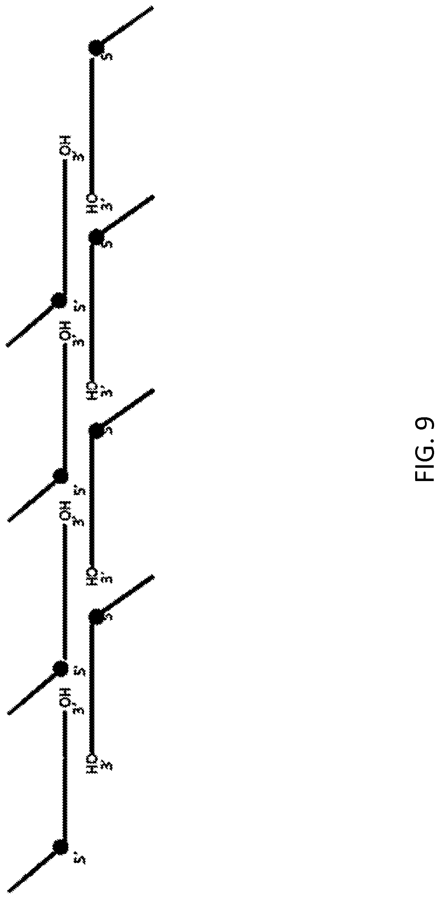

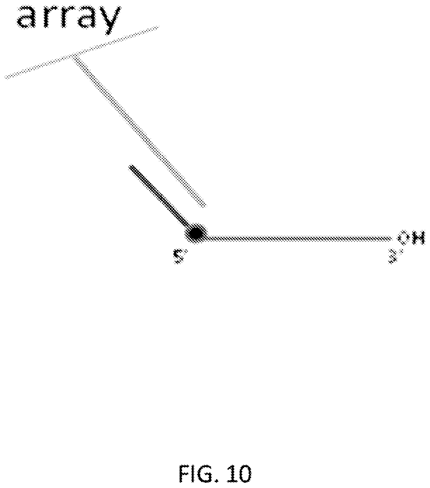

In some embodiments, the assembly sequences of each set comprise overlapping complementary sequences that can be ligated directly to one another after cleavage of the terminal indexer sequences. This arrangement of oligonucleotides is schematically illustrated in Fig. 9, where the overlapping complementary sequences (the "assembly sequences") are shown as being hybridized together as a duplex, the terminal indexer sequences are tails, and the cleavable linker is shown as a black dot between those sequences. As noted above, the terminal indexer sequences hybridize to a feature of an array, as illustrated in Fig. 10.

In some embodiments, the assembly sequences of each set comprise overlapping complementary sequences that can be assembled by polymerase chain assembly after cleavage of the terminal indexer sequences.

Also provided is an apparatus comprising: a planar support, a plurality of spatially distinct droplets on a surface of the planar support, and an immiscible liquid covering the droplets, wherein the apparatus comprises a plurality of reaction chambers defined by the droplets, and each reaction chamber comprises a different synthon.

## Claims

1. A method comprising:
(a) obtaining a mixture of multiple sets of oligonucleotides, wherein the oligonucleotides within each set each comprise a terminal indexer sequence and can be assembled to produce a synthon; and
(b) hybridizing the oligonucleotide mixture to a planar array, thereby spatially-separating the different sets of oligonucleotides from one another,
(c) contacting the array with a solution, thereby producing, for each feature bound by the oligonucleotides, a discrete droplet comprising one or more features,
(d) placing an immiscible liquid over the droplets, thereby producing, for each feature bound by the oligonucleotides, a discrete reaction chamber defined by a droplet,
(e) after step (d) incubating the array under conditions by which a synthon is assembled in each of the reaction chambers, and
wherein the terminal indexer sequence
- is a unique sequence at or near the end of said oligonucleotides in said mixture of multiple sets of oligonucleotides,
- is identical within each set of oligonucleotides, and
- is different from the terminal indexer sequence or its complement of any other set of oligonucleotides.

2. The method of claim 1, wherein the oligonucleotides
a) are single-stranded oligonucleotides; or
b) are single stranded and comprise a 3' hairpin.

3. The method of claim 1, wherein the mixture comprises double-stranded oligonucleotides.

4. The method of claim 2b, comprising: contacting the array with a solution comprising a polymerase and nucleotides, thereby extending the hairpin and producing, for each feature bound by the oligonucleotides, a set of double-stranded extension products.

5. The method of claim 1, wherein the oligonucleotides hybridize
a) directly to oligonucleotides that are immobilized on the array, or
b) via an adaptor to oligonucleotides that are immobilized on the array.

6. The method of claim 5b, wherein the method comprises: contacting the array with a solution comprising a polymerase and nucleotides, thereby extending the adaptor and producing, for each feature bound by the oligonucleotides, a set of double-stranded extension products.

7. The method of claim 1, wherein the droplets comprise double-stranded oligonucleotides or double-stranded extension products, and the solution comprises a Type lis restriction endonuclease, a DNA ligase and ATP, wherein the products of digestion of the double-stranded oligonucleotides or double-stranded extension products by the Type lis restriction endonuclease are ligated to one another in a defined order by the DNA ligase in the discrete reaction chambers, thereby producing a synthon.

8. The method of claim 1, wherein the oligonucleotides are single-stranded oligonucleotides and the method comprises:
cleaving the terminal indexer sequence from the oligonucleotides to release assembly sequences from at least some of the oligonucleotides; and
assembling the synthon from the assembly sequences by polymerase chain assembly or by ligation,
wherein said cleaving of the terminal indexer sequence from the oligonucleotide preferably comprises cleaving a photocleavable linkage.

9. The method of claim 1, wherein the oligonucleotides are double-stranded oligonucleotides that comprise staggered photocleavable or chemically cleavable linkages, and wherein the method comprises cleaving said staggered cleavable linkages using light or a chemical treatment to produce fragments that are ligatable to one another in order.

10. The method of claim 1, further comprising separating the synthons from the array.

11. A composition comprising multiple sets of oligonucleotides, wherein the oligonucleotides within each set comprise: (i) a terminal indexer sequence and (ii) an assembly sequence, wherein the terminal indexer sequence and the assembly sequence are separated by a photocleavable or chemically cleavable linker and the assembly sequences of each set of oligonucleotides can be assembled to produce a synthon, and
wherein the terminal indexer sequence
- is a unique sequence at or near the end of said oligonucleotides in said multiple sets of oligonucleotides,
- is identical within each set of oligonucleotides, and
- is different from the terminal indexer sequence or its complement of any other set of oligonucleotides.

12. The composition of claim 11, wherein the assembly sequences of each set comprise overlapping complementary sequences that can be
a) ligated directly to one another after cleavage of the terminal indexer sequences; or
b) assembled by polymerase chain assembly after cleavage of the terminal indexer sequences.

## Patentansprüche

1. Verfahren, umfassend:
(a) Erhalten einer Mischung aus mehreren Sätzen von Oligonukleotiden, wobei die Oligonukleotide innerhalb jedes Satzes jeweils eine terminale Index-Sequenz umfassen und zusammengefügt werden können, um ein Synthon zu erzeugen, und
(b) Hybridisieren der Oligonukleotidmischung an ein planares Feld, wodurch die verschiedenen Sätze von Oligonukleotiden räumlich voneinander getrennt werden,
(c) In-Kontakt-Bringen des Felds mit einer Lösung, wodurch für jedes durch die Oligonukleotide gebundene Merkmal ein einzelnes Tröpfchen erzeugt wird, das ein oder mehrere Merkmale umfasst,
(d) Aufbringen einer nicht mischbaren Flüssigkeit auf die Tröpfchen, wodurch für jedes an die Oligonukleotide gebundene Merkmal eine einzelne Reaktionskammer, die durch ein Tröpfchen definiert ist, erzeugt wird,
(e) Inkubieren des Felds nach Schritt (d) unter Bedingungen, durch die in jeder der Reaktionskammern ein Synthon zusammengesetzt wird, und
wobei die terminale Index-Sequenz
- eine eindeutige Sequenz am oder in der Nähe des Endes der Oligonukleotide in der Mischung aus mehreren Sätzen von Oligonukleotiden ist,
- innerhalb jedes Satzes von Oligonukleotiden identisch ist, und
- sich von der terminalen Index-Sequenz oder ihrem Komplement eines beliebigen anderen Satzes von Oligonukleotiden unterscheidet.

2. Verfahren nach Anspruch 1, wobei die Oligonukleotide
a) einzelsträngige Oligonukleotide sind oder
b) einzelsträngig sind und eine 3'-Haarnadel umfassen.

3. Verfahren nach Anspruch 1, wobei die Mischung doppelsträngige Oligonukleotide umfasst.

4. Verfahren nach Anspruch 2b, umfassend: In-Kontakt-Bringen des Felds mit einer Lösung, die eine Polymerase und Nukleotide umfasst, wodurch die Haarnadel verlängert wird und für jedes an die Oligonukleotide gebundene Merkmal ein Satz von doppelsträngigen Verlängerungsprodukten erzeugt wird.

5. Verfahren nach Anspruch 1, wobei die Oligonukleotide
a) direkt an Oligonukleotide, die auf dem Feld immobilisiert sind, oder
b) über einen Adaptor an Oligonukleotide, die auf dem Feld immobilisiert sind,
hybridisieren.

6. Verfahren nach Anspruch 5b, wobei das Verfahren umfasst: In-Kontakt-Bringen des Felds mit einer Lösung, die eine Polymerase und Nukleotide umfasst, wodurch der Adapter verlängert wird und für jedes Merkmal, das durch die Oligonukleotide gebunden wird, ein Satz von doppelsträngigen Verlängerungsprodukten erzeugt wird.

7. Verfahren nach Anspruch 1, wobei die Tröpfchen doppelsträngige Oligonukleotide oder doppelsträngige Verlängerungsprodukte umfassen und die Lösung eine Typ-IIs-Restriktionsendonuklease, eine DNA-Ligase und ATP umfasst, wobei die Produkte des Verdaus der doppelsträngigen Oligonukleotide oder der doppelsträngigen Verlängerungsprodukte durch die Typ-IIs-Restriktionsendonuklease in einer definierten Reihenfolge durch die DNA-Ligase in den einzelnen Reaktionskammern aneinander ligiert werden, wodurch ein Synthon erzeugt wird.

8. Verfahren nach Anspruch 1, wobei die Oligonukleotide einzelsträngige Oligonukleotide sind und das Verfahren umfasst:
Abspalten der terminalen Index-Sequenz von den Oligonukleotiden, um Assemblierungssequenzen von mindestens einigen der Oligonukleotide freizusetzen, und
Zusammensetzen des Synthons aus den Assemblierungssequenzen durch Polymerasekettenassemblierung oder durch Ligation,
wobei das Abspalten der terminalen Index-Sequenz von dem Oligonukleotid vorzugsweise das Spalten einer photospaltbaren Bindung umfasst.

9. Verfahren nach Anspruch 1, wobei die Oligonukleotide doppelsträngige Oligonukleotide sind, die gestaffelte photospaltbare oder chemisch spaltbare Bindungen umfassen, und wobei das Verfahren die Spaltung der gestaffelten spaltbaren Bindungen unter Verwendung von Licht oder einer chemischen Behandlung umfasst, um Fragmente zu erzeugen, die der Reihe nach miteinander ligierbar sind.

10. Verfahren nach Anspruch 1, ferner umfassend das Abtrennen der Synthons aus dem Feld.

11. Zusammensetzung, umfassend mehrere Sätze von Oligonukleotiden, wobei die Oligonukleotide innerhalb jedes Satzes umfassen: (i) eine terminale Index-Sequenz und (ii) eine Assemblierungssequenz, wobei die terminale Index-Sequenz und die Assemblierungssequenz durch einen photospaltbaren oder chemisch spaltbaren Linker getrennt sind und die Assemblierungssequenzen jedes Satzes von Oligonukleotiden zusammengesetzt werden können, um ein Synthon herzustellen, und
wobei die terminale Index-Sequenz
- eine eindeutige Sequenz am oder in der Nähe des Endes der Oligonukleotide in den mehreren Sätzen von Oligonukleotiden ist,
- innerhalb jedes Satzes von Oligonukleotiden identisch ist, und
- sich von der terminalen Index-Sequenz oder ihrem Komplement eines beliebigen anderen Satzes von Oligonukleotiden unterscheidet.

12. Zusammensetzung nach Anspruch 11, wobei die Assemblierungssequenzen eines jeden Satzes überlappende komplementäre Sequenzen umfassen, die
a) nach Abspaltung der terminalen Index-Sequenzen direkt aneinander ligiert werden oder
b) durch Polymerasekettenassemblierung nach Abspaltung der terminalen Index-Sequenzen zusammengesetzt werden
können.

## Revendications

1. Procédé comprenant :
(a) l'obtention d'un mélange de plusieurs ensembles d'oligonucléotides, les oligonucléotides au sein de chaque ensemble comprenant chacun une séquence d'indexation terminale et pouvant être assemblés pour produire un synthon ; et
(b) l'hybridation du mélange d'oligonucléotides sur un réseau plan, ce qui permet de séparer spatialement les différents ensembles d'oligonucléotides les uns des autres,
(c) la mise en contact du réseau avec une solution, ce qui permet de produire, pour chaque caractéristique liée par les oligonucléotides, une gouttelette distincte comprenant une ou plusieurs caractéristiques,
(d) le placement d'un liquide immiscible au-dessus les gouttelettes, ce qui permet de produire, pour chaque caractéristique liée par les oligonucléotides, une chambre de réaction distincte définie par une gouttelette,
(e) après l'étape (d), l'incubation du réseau dans des conditions par lesquelles un synthon est assemblé dans chacune des chambres de réaction, et
dans lequel la séquence d'indexation terminale
- est une séquence unique au niveau ou à proximité de l'extrémité desdits oligonucléotides dans ledit mélange de plusieurs ensembles d'oligonucléotides,
- est identique au sein de chaque ensemble d'oligonucléotides, et
- est différente de la séquence d'indexation terminale ou de son complément d'un quelconque autre ensemble d'oligonucléotides.

2. Procédé selon la revendication 1, dans lequel les oligonucléotides
a) sont des oligonucléotides monocaténaires ; ou
b) sont monocaténaires et comprennent une épingle à cheveux en 3'.

3. Procédé selon la revendication 1, dans lequel le mélange comprend des oligonucléotides bicaténaires.

4. Procédé selon la revendication 2b, comprenant : la mise en contact du réseau avec une solution comprenant une polymérase et des nucléotides, ce qui permet d'allonger l'épingle à cheveux et de produire, pour chaque caractéristique liée par les oligonucléotides, un ensemble de produits d'allongement bicaténaires.

5. Procédé selon la revendication 1, dans lequel les oligonucléotides s'hybrident
a) directement à des oligonucléotides qui sont immobilisés sur le réseau, ou
b) par le biais d'un adaptateur à des oligonucléotides qui sont immobilisés sur le réseau.

6. Procédé selon la revendication 5b, le procédé comprenant : la mise en contact du réseau avec une solution comprenant une polymérase et des nucléotides, ce qui permet d'allonger l'adaptateur et de produire, pour chaque caractéristique liée par les oligonucléotides, un ensemble de produits d'allongement bicaténaires.

7. Procédé selon la revendication 1, dans lequel les gouttelettes comprennent des oligonucléotides bicaténaires ou des produits d'allongement bicaténaires, et la solution comprend une endonucléase de restriction de type Ils, une ADN ligase et de l'ATP, les produits de la digestion des oligonucléotides bicaténaires ou des produits d'allongement bicaténaires par l'endonucléase de restriction de type Ils étant ligaturés l'un à l'autre dans un ordre défini par l'ADN ligase dans les chambres de réaction distinctes, ce qui permet de produire un synthon.

8. Procédé selon la revendication 1, dans lequel les oligonucléotides sont des oligonucléotides monocaténaires et le procédé comprend :
le clivage de la séquence d'indexation terminale des oligonucléotides afin de libérer des séquences d'assemblage à partir d'au moins certains des oligonucléotides ; et
l'assemblage du synthon à partir des séquences d'assemblage par assemblage en chaîne par polymérase ou par ligature,
ledit clivage de la séquence d'indexation terminale de l'oligonucléotide comprenant de préférence le clivage d'une liaison photoclivable.

9. Procédé selon la revendication 1, les oligonucléotides étant des oligonucléotides bicaténaires qui comprennent des jonctions photoclivables ou clivables chimiquement étagées, et le procédé comprenant le clivage desdites liaisons clivables étagées au moyen de lumière ou d'un traitement chimique pour produire des fragments qui peuvent être ligaturés les uns avec les autres dans l'ordre.

10. Procédé selon la revendication 1, comprenant en outre la séparation des synthons du réseau.

11. Composition comprenant plusieurs ensembles d'oligonucléotides, les oligonucléotides au sein de chaque ensemble comprenant : (i) une séquence d'indexation terminale et (ii) une séquence d'assemblage, la séquence d'indexation terminale et la séquence d'assemblage étant séparées par une séquence de liaison photoclivable ou clivable chimiquement et les séquences d'assemblage de chaque ensemble d'oligonucléotides peuvent être assemblées pour produire un synthon,
et la séquence d'indexation terminale
- étant une séquence unique au niveau ou à proximité de l'extrémité desdits oligonucléotides dans lesdits plusieurs ensembles d'oligonucléotides,
- étant identique au sein de chaque ensemble d'oligonucléotides, et
- étant différente de la séquence d'indexation terminale ou de son complément dans un quelconque autre ensemble d'oligonucléotides.

12. Composition selon la revendication 11, les séquences d'assemblage de chaque ensemble comprenant des séquences complémentaires chevauchantes qui peuvent être
a) ligaturées directement l'une à l'autre après clivage des séquences d'indexation terminales ; ou
b) assemblées par assemblage en chaîne par polymérase après clivage des séquences d'indexation terminales.
